# EUROPEAN PATENT SPECIFICATION

(11) **EP 1 370 219 B1**
(45) Date of publication and mention of the grant of the patent: **13.02.2008**
(21) Application number: 01973463.1
(22) Date of filing: 25.09.2001
(51) Int. Cl.: A61K 8/81, A61Q 1/10, A61Q 3/02, A61Q 5/00, A61Q 5/06, A61Q 19/00

(54) **USE OF ACRYLIC-BASED COPOLYMER COMPOSITIONS FOR COSMETIC AND PERSONAL CARE**
VERWENDUNG VON COPOLYMERZUSAMMENSETZUNGEN AUF ACRYLBASIS IN DER KOSMETIK UND FÜR DIE KÖRPERPFLEGE
UTILIZATION DE COMPOSITIONS DE COPOLYMERES ACRYLIQUES POUR SOINS COSMETIQUES ET PERSONNELS

(30) Priority: 25.10.2000 US 696468; 22.06.2001 US 887751
(43) Date of publication of application: 17.12.2003
(73) Proprietor: 3M Innovative Properties Company, St. Paul, MN 55133-3427 (US)
(72) Inventor: KANTNER, Steven, S., Saint Paul, MN 55133-3427 (US); EVERAERTS, Albert, I., Saint Paul, MN 55133-3427 (US); NGUYEN, Lang, N., Saint Paul, MN 55133-3427 (US)
(74) Representative: Meyers, Hans-Wilhelm
(86) International application number: PCT/US2001/029883
(87) International publication number: WO 2002/034218

(56) References cited:
- EP-A- 0 985 405
- US-A- 5 662 892
- US-A- 5 672 576
- US-A- 6 126 929

## Description

### Technical Field

The present invention relates to the use of compositions for cosmetic and personal care, such as skin and nails. In particular, the composition is an aqueous, acrylic-based copolymer emulsion or dispersion that dries rapidly to form a flexible, non-sticky film.

### Background

Polymers have been used to minimize or prevent the transfer of makeup or the wash-off of sunscreen. Generally, these polymers are hydrophobic and obtain their hydrophobicity from long chain alkenes (*e.g.*, U.S. Patent Nos. 5,026,540; 5,171,807; 5,219,559; 5,516,508; 5,518,712; and 5,547,659) and from long chain alkyl (meth)acrylates (*e.g.*, U.S. Patent Nos. 4,172,122 and 4,552,755). Also disclosed as having utility for the above stated purpose include silicone pressure sensitive adhesives (*e.g.,* U.S. Patent No. 5,460,804), styrene-ethylene-propylene block copolymers (*e.g*., U.S. Patent No. 6,060,072), or polymers containing long chain vinyl or allyl ester comonomers (*e.g.*, U.S. Reissue 29,871). The polymers listed thus far generally have a low glass transition temperature (T_{g}) and thus can often leave skin with an undesirable sticky or tacky feel. They may also have poor cohesive strength giving a greasy feel and causing staining. Makeup or sunscreen formulated using these polymers may be difficult to apply smoothly and uniformly, due to the polymers' draginess thus leaving a leaden skin feel. Such a feel is undesirable, particularly in lipstick.

In another approach, some skilled in the art have used high T_{g} polymers for cosmetic applications, *e.g.*, in hair styling aids and in nail lacquers. In hair styling compositions, the high T_{g} polymers generally are glassy due to the high levels of polar monomers used. Such monomers contain acid, amide, amine, or hydroxyl functionality, as described in U.S. Patent No. 5,019,377. The high level of polar monomers in the polymer can detract the polymer's ability to provide water resistance in cosmetic and sunscreen formulations.

In yet another approach, up to 20% of hydrophobic high T_{g} monomers, such as isobornyl acrylate, have been used to prepare a terpolymer with polar vinyl ester and alkyl maleate half ester comonomers (*e.g.,* EP 299,025 and WO 98/51266) to provide stability during the suspension polymerization, to ensure solubility in commercial alcohol carriers, and to minimize tack.

Nail lacquers are generally applied from organic solvent and hence hydrophobic high T_{g} polymers, such as nitrocellulose, are commonly used. U.S. Patent No. 4,762,703 (Abrutyn) discloses an anhydrous nail lacquer composition containing 10 to 40% by weight of a copolymer which is the reaction product of 5 to 30% by weight of diacetone acrylamide with 60 to 95% by weight of (meth)acrylate esters of (a) 5 to 48% straight chain alcohols, (b) 5 to 60% cyclic alcohols, (c) 1 to 25% higher alkyl alcohols, and (d) 1 to 30% alkoxy or aryloxy alkyl alcohols. The use of an aqueous carrier, solvent or vehicle component is not disclosed, nor is the use of these materials in cosmetic and sunscreen compositions for skin and hair.

U.S. Patent No. 5,662,892 (Bolich, Jr. et al.) discloses personal care compositions, in particular hair care compositions containing hydrophobic, linear, random copolymers and a hydrophobic, volatile, branched hydrocarbon solvent for the copolymer. The linear copolymers are formed from the random copolymerization of A monomer units and B monomer units. The A monomer units are one or more hydrophobic monomer units that would form a homopolymer having a T_{g} of at least 90° C. The B monomer units are one or more hydrophobic monomers that would form a homopolymer having a T_{g} of less than about 25° C. The copolymers, when dried to form a film, have a T_{g} of at least about 30° C, and tend to have low stickiness and provide good style hold. The linear copolymer is soluble in the branched chain hydrocarbon solvent. But, the hydrocarbon solvent is insoluble in aqueous carriers of the composition. Upon drying, the preferred hydrocarbon solvents help to obtain a smoother polymer film. Other advantages of using the hydrocarbon solvent were discussed. See column 7, lines 10 to 25.

While the technology discussed thus far may be useful for cosmetic applications, other compositions are sought.

### Summary

The present invention provides for the use of compositions for cosmetic and personal care applications, where compositions contain non-sticky hydrophobic polymers in an aqueous carrier, solvent or vehicle. Advantageously, such compositions can be used for skin and nails. The compositions provide improved resistance against abrasion, transfer, water, perspiration, and humidity while having excellent gloss, feel, and self adhesion.

The use of the present invention is defined in claim 1. Blends of two or more disclosed copolymers are also useful. Optionally, the copolymer can have up to about 20 weight percent of a hydrophilic monomer (conveniently labeled as a third monomer). The weight percentages of the first, second, and, if used, third monomers, are based on the total weight of the monomers used.

The term "(meth)acrylate" is used to mean both acrylate and methacrylate. The term "dispersion" means generally a two phase system where one phase contains discrete particles distributed throughout a bulk substance, the particles being the disperse or internal phase, and the bulk substance the continuous or external phase. In this invention, the continuous phase is the aqueous phase and at least a portion of the polymer exists as the discrete particle. Dispersions are possible through the use of certain components that are insoluble in the water system. By "dispersion," it is also meant that not necessarily the entire polymer needs to be water insoluble; some of the polymer can be soluble in the water mixture. It is desirable that the dispersion remains stable under ambient conditions. Preferred dispersions are stable at room temperature for more than 30 days, preferably more than 90 days, more preferably for more than 180 days, and most preferably for more than 360 days. The term "blend" is used to mean a mixture of two or more polymers that differ in the ratio of monomer components, the chemical structure of the monomer components, the monomer sequence distribution, and/or the polymer's molecular weight distribution.

Some inventive compositions, in film form, possess "self adhesion" properties because they preferentially adhere to themselves or a chemically similar material under pressure or force without the need for significantly elevated temperatures (*e.g.*, without the need for temperatures above about 50° C). Preferred compositions of the invention exhibit self adhesion properties immediately upon contact to itself at room temperature (about 20° to 30° C). As used in the previous sentence, the term "immediately" means less than a few minutes, *e.g.*, about five minutes, preferably less than one minute, more preferably less than 30 seconds, depending on the application.

An advantage of the inventive composition is its ability to form hydrophobic films making it useful in cosmetic and personal care applications. Such applications require some amount of water resistance, transfer resistance, or substantivity to skin or nails. Cosmetic applications are mascara, foundation, rouge, face powder, eyeliner, eyeshadow, nail polish, and lipstick, *i.e.,* color cosmetics. Personal care applications are insect repellent, skin moisturizer, skin cream, body lotion, body spray, and sunscreen. In one cosmetic or personal care embodiment, the composition comprises less than 50 weight percent of the copolymer, based on the total composition weight.

### Detailed Description of the Invention

As described above the composition contains an aqueous carrier, solvent or vehicle and at least one copolymer having a first monomer, a second monomer, and optionally a third monomer. The amount and nature of each component is chosen such that, upon drying, the composition forms a flexible, non-sticky film having good cohesive strength. In some embodiments, the composition also possesses self-adhesion. Each of the components constituting the composition is discussed in detail below. As used herein, "copolymers" can be produced from a single monomer or a single homopolymer, from two or more monomers or from a polymer and one or more monomers.

### First Monomer

The first monomer is hydrophobic in nature. It constitutes from about 10 to 85 weight percent of the total amount of monomer used. The first monomer is a (meth)acrylate ester of C₄ to C₁₈ straight and/or branched chain alkyl alcohol. Preferred first monomers include, *e.g.*, isooctyl (meth)acrylate, n-butyl (meth)acrylate, isobutyl acrylate, t-butyl (meth)acrylate, 2-methylbutyl acrylate, 2-ethylhexyl (meth)acrylate, n-octyl (meth)acrylate, isononyl (meth)acrylate, lauryl (meth)acrylate, octadecyl (meth)acrylate, and mixtures thereof. Particularly preferred first monomers include 2-ethylhexyl acrylate, n-butyl acrylate, isooctyl acrylate, 2-methylbutyl acrylate, and mixtures thereof.

### Second Monomer

The second monomer is also hydrophobic in nature and, generally speaking, has a higher T_{g} than the first monomer. The second monomer constitutes from about 10 to 70 weight percent of the total amount of monomer used. It is a (meth)acrylate ester of a saturated or unsaturated cyclic alcohol containing 6 to 20 carbon atoms. Preferred second monomers include, *e.g.*, monofunctional acrylate or methacrylate esters of (1) bridged cycloalkyl alcohols having at least six carbon atoms and (2) aromatic alcohols. The cycloalkyl and aromatic groups may be substituted by C₁ to C₆ alkyl, halogen, cyano groups and the like. Particularly preferred second monomers include bicyclo[2.2.1]heptyl (meth)acrylate; adamantyl (meth)acrylate; 3,5-dimethyladamantyl (meth)acrylate; isobornyl (meth)acrylate; tolyl (meth)acrylate; phenyl (meth)acrylate; t-butylphenyl (meth)acrylate; 2-naphthyl (meth)acrylate; benzyl methacrylate; cyclohexyl methacrylate; menthyl methacrylate; 3,3,5-trimethylcyclohexyl methacrylate; dicyclopentenyl (meth)acrylate; 2-(dicyclopentenyloxy)ethyl (meth)acrylate; and mixtures thereof.

### Optional Third Monomer

In some embodiments of the invention, the copolymer further contains a third monomer, which constitutes up to about 20 weight percent of the total amount of monomer used. The third monomer is a hydrophilic monomer. Incorporation of hydrophilic monomer can improve adhesion, allow for removal with soap or shampoo, and provide stabilization to allow dispersion of the polymer into water. Removability is typically not desired in applications where long-lasting effect is desired, such as sunscreens, mascara, and lipstick.

The hydrophilic monomers suitable for use in this invention are those having hydroxyl, ether, amide, amine, and carboxylic, sulfonic or phosphonic acid functionality. Representative examples include (meth)acrylamide; 2-ethoxyethyl (meth)acrylate; mono (meth)acrylates of polyethylene glycol monoethers; N-vinyl-2-pyrrolidone; N-vinyl formamide; N-vinyl acetamide; 2-hydroxyethyl (meth)acrylate; hydroxypropyl acrylate; vinyl pyridine; N,N-diethylaminoethyl methacrylate; N,N-dimethylaminoethyl (meth)acrylate; N-t-butylaminoethyl acrylate, acrylic acid, methacrylic acid, itaconic acid, maleic acid, fumaric acid, vinyl benzoic acid, 2-carboxyethyl acrylate, 2-sulfoethyl (meth)acrylate, and 4-vinyl phenyl phosphonic acid. Preferred hydrophilic monomers are acrylic acid, methacrylic acid, N-vinyl-2-pyrrolidone and mixtures thereof. The amount of hydrophilic monomer preferably does not exceed about 20%, more preferably about 10% of the total weight of all monomers, such that excessive hydrophilicity is avoided. Those skilled in the art, however, will recognize that a monomer such as 2-ethoxyethyl methacrylate is less hydrophilic than a monomer such as acrylic acid and hence can be used in higher amount without imparting excessive hydrophilicity.

The copolymer may include other monomers to improve performance, reduce cost, or for other purposes, provided that such monomers are used in an amount that does not render the composition hydrophilic or tacky. Examples of such other monomers include vinyl esters, vinyl chloride, vinylidene chloride, styrene, (meth)acrylate esters of C₁ to C₃ alkyl alcohols, macromolecular monomers such as monoacrylic functional polystyrene and polydimethylsiloxane, and the like.

### Blends

The composition can comprise a blend of two or more copolymers. Among other techniques, these blends can be formed (1) by mixing two or more aqueous dispersions or emulsions or (2) in a multistage, sequential polymerization process where a second polymer is generated in the presence of a first polymer. Blending provides another option allowing modification of the final properties. For example, a blend of a given copolymer composition having a low molecular weight distribution with the same copolymer composition having a higher molecular weight distribution can give a dispersion or emulsion with improved film forming characteristics while maintaining good cohesion in the final film.

### Aqueous Carrier

The composition takes the form of an emulsion or dispersion in an aqueous carrier. The carriers include water, water miscible solvents, such as lower alcohols, *e.g.*, C₁ to C₄ branched or straight chain aliphatic alcohol, and combinations thereof. The preferred water miscible solvents are ethanol, n-propanol, and 2-propanol (IPA). Preferably the solvent to water ratio, when solvent is used, is 20:80 to 90:10 weight/weight, and more preferably the ratio is 30:70 to 85:15. In general, higher water miscible solvent amounts will result in a composition that exhibits faster dry times.

The solvent system may also comprise additional solvents. For example, other rapid evaporating solvents may be used, such as hexamethyldisiloxane (HMDS); cyclic silicones (D₄ and D₅); C₄-C₁₀ alkanes including isoparafins such as Permethyl 97A and Isopar C; acetone; hydrofluoroethers (HFEs) and the like. Certain HFEs, such as HFE 7100, have the added benefit in certain applications. When it is added to hydro-alcohol mixtures in levels above about 15 to 25% by weight, the composition becomes non-flammable.

### Cosmetic Compositions

The emulsion or dispersion is useful by itself for cosmetic purposes without addition of other materials, for instance as a skin barrier, or clear nail polish. It can also be formulated with other ingredients known to the cosmetic industry to give cosmetic compositions containing an aqueous component. Such ingredients include emollients, humectants, other film forming polymers, propellants, pigments, dyes, buffers, organic and inorganic suspending and thickening agents, waxes, surfactants and cosurfactants, plasticizers, preservatives, flavoring agents, perfumes, and active ingredients including sunscreen agents, insect repellents, vitamins, herbal extracts, antiperspirant and deodorant agents, skin bleaching or coloring agents, depilating agents, antifungal and antimicrobial agents, antiacne agents, astringents, and corn, callus, and wart removers.

### Methods of Making the Copolymer

The copolymers used in the present invention may be prepared using emulsion polymerization, solution polymerization followed by an inversion step, and suspension polymerization. The methods use initiators that, through various techniques, are decomposed to form free radicals. Once in their radical form, the initiators react with the monomers starting the polymerization process. The initiators are often called "free radical initiators." Various decomposition methods of the initiators are discussed first, followed by a description of the emulsion, solution, and suspension polymerization methods.

The initiator can be decomposed homolytically to form free radicals. Homolytic decomposition of the initiator can be induced by using heat energy (thermolysis), using light energy (photolysis), or using appropriate catalysts. Light energy can be supplied by means of visible or ultraviolet sources, including low intensity fluorescent black light lamps, medium pressure mercury arc lamps, and germicidal mercury lamps.

Catalyst induced homolytic decomposition of the initiator typically involves an electron transfer mechanism resulting in a reduction-oxidation (redox) reaction. This redox method of initiation is described in Elias, Chapter 20 (detailed below). Initiators such as persulfates, peroxides, and hydroperoxides are more susceptible to this type of decomposition. Useful catalysts include, but are not limited to, (1) amines, (2) metal ions used in combination with peroxide or hydroperoxide initiators, and (3) bisulfite or mercapto-based compounds used in combination with persulfate initiators.

Presently, preferred methods of initiation comprise thermolysis or catalysis. Thermolysis has an additional advantage in that it provides ease of control of the reaction rate and exotherm.

Useful initiators are described in Chapters 20 & 21 Macromolecules, Vol. 2, 2nd Ed., H. G. Elias, Plenum Press, 1984, New York. Useful thermal initiators include, but are not limited to, the following: (1) azo compounds such as 2,2-azo-bis-(isobutyronitrile), dimethyl 2,2'-azo-bis-isobutyrate, azo-bis-(diphenyl methane), 4-4'-azo-bis-(4-cyanopentanoic acid); (2) peroxides such as benzoyl peroxide, cumyl peroxide, tert-butyl peroxide, cyclohexanone peroxide, glutaric acid peroxide, lauroyl peroxide, methyl ethyl ketone peroxide; (3) hydrogen peroxide and hydroperoxides such as tert-butyl hydroperoxide and cumene hydroperoxide; (4) peracids such as peracetic acid and perbenzoic acid; potassium persulfate; ammonium persulfate; and (5) peresters such as diisopropyl percarbonate.

Useful photochemical initiators include but are not limited to benzoin ethers such as diethoxyacetophenone, oximino-ketones, acylphosphine oxides, diaryl ketones such as benzophenone and 2-isopropyl thioxanthone, benzil and quinone derivatives, and 3-ketocoumarins as described by S. P. Pappas, J. Rad. Cur., July 1987, p.6.

### Emulsion polymerization

The copolymers used in the present invention can be made by emulsion polymerization. In general, it is a process where the monomers are dispersed in a continuous phase (typically water) with the aid of an emulsifier and polymerized with the free-radical initiators described above. Other components that are often used in this process include stabilizers (*e.g.*, copolymerizable surfactants), chain transfer agents for minimizing and/or controlling the polymer molecular weight, and catalysts. The product of this type of polymerization is typically a colloidal dispersion of the polymer particles, often referred to as "latex." In one preferred emulsion polymerization process, a redox chemistry catalyst, such as sodium metabisulfite, used in combination with potassium persulfate initiator and ferrous sulfate heptahydrate, is used to start the polymerization at or near room temperature. Typically, the copolymer particle size is less than one micrometer, preferably less than 0.5 micrometer.

Emulsion polymerization can be carried out in several different processes. For example, in a batch process the components are charged into the reactor at or near the beginning. In a semi-continuous process, a portion of the monomer composition is initially polymerized to form a "seed" and the remaining monomer composition is metered in and reacted over an extended time. In one exemplary multistage process, a seed polymer of one monomer composition (or one molecular weight distribution) is used to nucleate the polymerization of a second monomer composition (or the same composition with a different molecular weight distribution) forming a heterogeneous polymer particle. These emulsion polymerization techniques are well known by those skilled in the art and are widely used in industry.

### Solution polymerization and inversion

The copolymers used in the present invention can be made by solution polymerization followed by an inversion step. In one illustrative solution polymerization method, the monomers and suitable inert solvents are charged into a reaction vessel. The monomers and the resultant copolymers are soluble in the solvent. After the monomers are charged, an initiator, preferably a thermal free radical initiator is added. The vessel is purged with nitrogen to create an inert atmosphere. The reaction is allowed to proceed, typically using elevated temperatures, to achieve a desired conversion of the monomers to the copolymer. In solution polymerization, preferably the initiator used comprises a thermally decomposed azo or peroxide compound for reasons of solubility and control of the reaction rate.

Suitable solvents for solution polymerizations include but are not limited to (1) esters such as ethyl acetate and butyl acetate; (2) ketones such as methyl ethyl ketone and acetone; (3) alcohols such as methanol and ethanol; (4) aliphatic and aromatic hydrocarbons; and mixtures of one or more of these. The solvent, however, may be any substance which is liquid in a temperature range of about -10° C to 50° C, does not interfere with the energy source or catalyst used to dissociate the initiator to form free radicals, is inert to the reactants and product, and will not otherwise adversely affect the reaction. The amount of solvent, when used, is generally about 30 to 80 percent by weight based on the total weight of the reactants and solvent. Preferably, the amount of solvent ranges from about 40% to 65% by weight, based upon the total weight of the reactants and solvent, to yield fast reaction times.

Copolymers prepared by solution polymerization can be inverted to yield dispersions of small average particle size, typically less than about one micrometer, preferably less than about 0.5 micrometer. Inversion of copolymers can occur in aqueous carrier or aqueous solvent provided that (1) they contain ionic functionality or (2) they contain acidic or basic functionality, which on neutralization yields ionic functionality.

Copolymers containing acidic functionality are obtained by copolymerizing acidic monomers. Suitable acidic monomers include those containing carboxylic acid functionality such as acrylic acid, methacrylic acid, itaconic acid, *etc.;* those containing sulfonic acid functionality such as 2-sulfoethyl methacrylate; and those containing phosphonic acid functionality. Preferred acidic monomers include acrylic acid and methacrylic acid.

Copolymers containing basic functionality are obtained by copolymerizing basic monomers. Suitable basic monomers include those containing amine functionality such as vinyl pyridine; N,N-diethylaminoethyl (meth)acrylate; N,N-dimethylaminoethyl (meth)acrylate; and N-t-butylaminoethyl acrylate. Preferred basic monomers include N,N-dimethylaminoethyl (meth)acrylate.

In order to achieve water compatibility or dispersibility, a certain minimum ionic content in the copolymer is required. The exact amount varies with the particular polymer formulation, the molecular weight of the copolymer, and other features of the individual copolymer. However, the addition of ionic groups, while increasing water miscibility, can negatively affect polymer properties, in particular the water, perspiration, and humidity resistance that the copolymer imparts to cosmetic formulations. It is therefore preferred that the ionic content either be kept to the minimum amount required to yield stable aqueous dispersions while maintaining other desirable properties, or that the ionic content introduced to achieve water dispersibility be non-permanent in nature. As described below, this non-permanent feature is achieved by using a volatile, weak acid or base in the neutralization technique, thereby allowing the polymer to revert to its original state on coating and drying. Generally a minimum of about 2% by weight of ionic content will yield a stable dispersion. The amount of the ionic group includes only the simplest of constructions, *i.e.*, the monomer from which the ionic group is derived plus the base or acid used to neutralize it, as the molecular weight of the ion. Preferred copolymers contain about 4% ionic content. Copolymers with permanent ionic content of over about 15% are too hydrophilic for use in most skin applications.

Preferably the copolymer is prepared in a water-miscible solvent which has a boiling point below 100° C such as acetone or methyl ethyl ketone. Alternatively, a non-water-miscible polymerization solvent such as ethyl acetate may be used. The non-water-miscible polymerization solvent may be removed from the copolymer by using a rotary evaporator. The resulting copolymer can then be dissolved in a water-miscible solvent such as those described above or mixtures including isopropanol, methanol, ethanol, and tetrahydrofuran.

The resulting solutions are added with stirring to an aqueous solution of a base, (in the case of copolymers containing acidic functionality), or an acid (in the case of copolymers containing basic functionality). Alternatively, the base or acid can be added to the polymer solution prior to adding water or adding to water. Suitable bases include (1) ammonia and organic amines, such as aminomethyl propanol, triethyl amine, triethanol amine, methyl amine, morpholine, and (2) metal hydroxides, oxides, and carbonates, *etc*. Suitable acids include (1) carboxylic acids such as acetic acid, and (2) mineral acids, such as HCl. In the case of a volatile weak base (*e.g.*, ammonia) or acid (*e.g.*, acetic acid), the ionic group formed (an ammonium carboxylate) is non-permanent in nature. For example, for an acrylic acid containing polymer neutralized with aqueous ammonia, the polymer remains as the ammonium acrylate derivative when dispersed in water, but is thought to revert to its original free acid state as the coating dries on the surface. This is because there is an equilibrium between the neutralized and free acid which is shifted towards the free acid as the ammonia is driven off on drying. Acid or base at less than an equivalent is preferably used, more preferably at slightly less than an equivalent, to ensure near neutral pH and thus providing the lowest potential for skin irritation.

### Suspension Polymerization

The copolymers used in the present invention can be made by a suspension polymerization method in the absence of surfactants. Instead, colloidal silica in combination with a promoter is used as the stabilizer. Using this process, surfactant-free copolymers can be obtained with a relatively narrow particle size distribution. The preferred method involves making a monomer premix comprising the first, second, and optionally third monomer. The premix is combined with a water phase, preferably deionized water, containing colloidal silica, and a promoter. Amphiphilic polymers represent one class of useful promoters.

The pH of the mixture is adjusted so as to be in the range of 3 to 11, preferably in the range of 4 to 6, without coagulation of the particles. For certain monomers, the initial pH of the mixture can be as low as about 2.5. This pH is low enough for the colloidal silica to stabilize the monomer droplet, but the final product may contain a small amount of coagulum. Similar observation can be made at very high pH. It has been observed that when the mixture is treated with ammonia or hydrochloric acid to about pH 4 to 6, the reaction is more stable and the final product is basically free of coagulum.

The mixture is exposed to high shear, such as that capable in a Waring^{™} blender, to break the monomer droplets down to a diameter size of 1 micrometer or less. The shearing action is then reduced to a lower agitation (or temporarily stopped) to allow for the partial coalescence of the small droplets and formation of a suspension. Initiator is added. The silica-promoter mixture stabilizes the droplets and limits their coalescence yielding very uniform, and sometimes nearly monodisperse particles. The suspension polymerization is completed under moderate agitation and a stable, aqueous dispersion of acrylic particles is obtained.

The above described suspension polymerization has several advantages. For example, the method yields a copolymer with a narrow distribution of mean particle size and limited coalescence. When coalescence is present, the particles tend to migrate towards one another and can form large masses. Coalescence hampers the handling and transportation of the particles and thus is undesirable. The particles are sterically stabilized by the colloidal silica.

Also, the method allows for copolymers that withstand freezing temperatures, allowing them to be redispersed after thawing. It has been discovered that the copolymer is stable, *i.e.*, does not coalesce when the same volume of alcohol (methanol or isopropanol) and water is used in the dispersion.

### Examples

The following examples further illustrate various specific features, advantages, and other details of the invention. The particular materials and amounts recited in these examples, as well as other conditions and details, should not be construed in a manner that would unduly limit the scope of this invention. Percentages given are by weight, unless otherwise specified.

### Test Methods

Test methods used to evaluate flexibility (or brittleness) and stickiness (or tack) of coatings prepared from the materials described below are industry standard tests as further described below.

### Flexibility

The flexibility of each coating was assessed using ASTM D 4338-97, "Standard Test Method for Flexibility Determination of Supported Adhesive Films by Mandrel Bend." The coated polyester was folded with adhesive side out over a 0.125 inch (3.2 mm) rod and the development of cracks, fracture, or crazing noted as a failure.

### Tack

The tack of each coating was assessed using ASTM D 2979-95, "Standard Test Method for Pressure-Sensitive Tack of Adhesives Using an Inverted Probe Machine." A Polyken Probe Tack Series 400 Tester was used with a dwell time of one second, a contact and removal speed of one centimeter per second, and a annular ring weighing 19.8 grams. The 5 millimeter stainless steel probe was cleaned with isopropanol between samples and five replicates were run on each coating and averaged to give the results reported in Table II. Materials useful in formulating cosmetic compositions have a tack value of less than 50 grams, preferably less than 30 grams, most preferably equal to 0 grams. Useful materials will also possess high enough cohesive strength so that they do not cohesively fail, thereby transferring residue to the probe.

### Glass Transition Temperature

The glass transition temperature (T_{g}) of each polymer was assessed using a Perkin-Elmer Model DSC 7 differential scanning calorimeter. Samples were dried in aluminum tins at 105° C for 30 minutes. Samples, ranging from six to ten milligrams, were scanned heating from -70° to 150° C at 20° C/minute. After holding at 150° C for 1 minute, the sample was cooled to -70° C at 40° C/minute, then scanned a second time to 150° C at 20° C/minute. The extrapolated midpoint of the inflection in this second heat is reported as the T_{g}.

### Preparation of Coatings

Coatings of the examples were prepared on 0.0015 inch (38 micrometer, µm) thick polyester film using a knife coater yielding 0.0015 inch (38 µm) thick coatings after drying for 10 minutes at 70° C in a forced air oven. These coatings were conditioned for 24 hours at 22° C and 50% relative humidity prior to testing.

### Examples 1 to 8, Comparative Examples A to D

### Copolymers made by batch emulsion polymerization

Into a one liter Mortonized split resin flask was charged 100 grams of monomers (detailed in Table I below, all monomer amounts listed in grams), 80 milligrams of carbon tetrabromide, 124.7 grams of deionized water, 200 milligrams of potassium persulfate, 64 milligrams of sodium metabisulfite, 1 gram of sodium dodecyl benzene sulfonate, and 2.5 grams of Mazon SAM 211 alkylene polyalkoxy ammonium sulfate copolymerizable surfactant (available from PPG Industries, Pittsburgh, PA). The head was placed on the flask and a thermocouple, nitrogen inlet, and mechanical stirrer attached. The headspace was swept with nitrogen at 1 liter per minute while heating the contents with infra red lamps to about 30° C and stirring at 250 rpm. About 1 gram of a solution of 28 milligrams ferrous sulfate heptahydrate in 50 grams deionized water was charged, the flask sealed, and a vacuum pulled on the flask three times, breaking it each time with nitrogen. After 15 or 20 minutes an exotherm is noted which peaks 20 to 25 minutes later at 55° to 65° C. Reactor temperature is increased to about 75° C and held for one hour, and then the resulting latex was filtered through doubled over cheesecloth into a jar. In all cases moderate levels of coagulum were noted around the thermocouple and stirring paddle.

### Comparative Example E

An acrylate terpolymer such as the ones disclosed in U.S. Patent Nos. 4,172,122 and 4,552,755 is made as follows.

Into a one liter bottle was charged 280 grams of ethyl acetate, 94.6 grams of isooctyl acrylate, 110 grams of stearyl methacrylate, 15.4 g of acrylic acid, and 0.77 grams of 2,2'-azobis(2-methylbutyronitrile), sold by E.I. du Pont de Nemours & Co., Wilmington, DE, as Vazo^{™} 67. The resulting solution was purged for about 5 minutes with nitrogen at 5 liters per minute, sealed, and tumbled in a water bath at about 60° C for about 63 hours. A hazy, moderately thick solution resulted.

### Comparative Example F

A 40% solids solution of GANEX V216 (available from ISP, and believed to be a N-vinyl pyrrolidone/hexadecene copolymer) was prepared by dissolving about 4 grams of GANEX V216 in about 6 grams of ethyl acetate with gentle heating.

**Table I: Monomer Charges Used for Emulsion Polymerization**

| **Example** | **2-EHA** | **IBOA** | **AA** | **MAA** | **T_{g} (°C)** | **Tack (g)** | **Flexibility** |
|---|---|---|---|---|---|---|---|
| Comparative A | 70 | 25 | 0 | 5 | -39 | 59 | pass |
| Comparative B | 60 | 35 | 5 | 0 | -26 | 383 | pass |
| Comparative C | 55 | 40 | 5 | 0 | -19 | 274 | pass |
| Comparative D | 25 | 70 | 0 | 5 | 39 | 0 | fail |
| Comparative E | NA | NA | NA | NA | NA | 316 | pass |
| Comparative F | NA | NA | NA | NA | NA | NA | pass |
| 1 | 60 | 35 | 0 | 5 | -26 | 0 | pass |
| 2 | 55 | 40 | 0 | 5 | -19 | 0 | pass |
| 3 | 50 | 45 | 5 | 0 | -13 | 29 | pass |
| 4 | 50 | 45 | 0 | 5 | -9 | 0 | pass |
| 5 | 50 | 40 | 0 | 10 | -13 | 0 | pass |
| 6 | 45 | 50 | 5 | 0 | -4 | 0 | pass |
| 7 | 45 | 50 | 0 | 5 | -3 | 0 | pass |
| 8 | 35 | 60 | 0 | 5 | 15 | 0 | pass |

| | | | | | | | |
|---|---|---|---|---|---|---|---|
| 2-EHA = 2-ethylhexyl acrylate IBOA = isobornyl acrylate AA = acrylic acid MAA = methacrylic acid NA = not applicable | | | | | | | |

As the data in Table I shows, Comparative Examples A, B, C, and E had unacceptably high tack values even though they passed the flexibility test. Comparative Example D failed the flexibility test because it cracked and flaked off the polyester film. Useful compositions possess high enough cohesive strength to pass tack testing and not transfer residue to the test probe. Comparative Example F had too low a cohesive strength. The T_{g} was not a necessary requirement for determining tack, *i.e.*, T_{g} was not a good indicator of whether a sample passed the tack test. But, to pass the mandrel test, the T_{g} should be below about 35° C.

### Example 9

### Tetrapolymer made by semi-continuous emulsion polymerization

A solution of 1.0 grams carbon tetrabromide was prepared in a mixture of 275 grams 2-EHA, 200 grams IBOA, 12.5 grams MAA and 12.5 grams AA yielding 500 grams of a monomer solution containing 55/40/2.5/2.5 parts 2-EHA/IOBA/MAAIAA. Of the total monomer solution, 50 grams was charged into a two-liter split resin flask along with 390 grams of deionized water and 0.5 gram of sodium dodecyl benzene sulfonate. The head was placed on the flask and a thermocouple, nitrogen inlet, and mechanical stirrer attached. The contents were heated with infrared lamps to about 60° C while stirring at 350 rpm. A solution of 1.25 grams potassium persulfate in 20 grams deionized water was charged, the flask sealed, and a vacuum pulled on the flask four times, breaking it each time with nitrogen. The flask was held at 60° C for 20 minutes, then heated to 80° C over 10 minutes to yield a seed polymer. A pre-emulsion of the remaining 450 grams of the monomer solution was prepared by charging a solution of 4.5 grams of sodium dodecyl benzene sulfonate in 201 grams of deionized water to it and stirring under nitrogen. This pre-emulsion was added dropwise to the two liter split resin flask containing the seed polymer at a rate of 6 grams per minute. The addition took almost 2 hours. After the addition, the stirring rate was reduced to 200 rpm and the reaction held at 80° C for two hours, then the resulting latex was filtered through doubled over cheesecloth into a jar. Low levels of coagulum were noted around the thermocouple and stirring paddle.

### Examples 10 and 11

### Terpolymers made by semi-continuous emulsion polymerization

Using the procedure of Example 9, monomer solutions of either 300 grams 2-EHA, 175 grams IBOA, 25 grams MAA, and 1 gram carbon tetrabromide (Example 10) or 250 grams 2-EHA, 225 grams IBOA, 25 grams AA, and 1 gram carbon tetrabromide (Example 11) were polymerized. The monomer solution of Example 10 contained 60/35/5 parts 2-EHA/IBOA/MAA. The monomer solution of Example 11 contained 50/45/5 parts 2-EHA/IBOA/AA.

### Example 12

### Heterogeneous copolymer made by sequential emulsion polymerization

A first monomer solution of 0.5 grams carbon tetrabromide was prepared in a mixture of 150 grams 2-EHA, 87.5 grams IBOA, and 12.5 grams MAA. Of the first monomer solution, 50 grams was charged into a two-liter split resin flask along with 390 grams of deionized water and 0.5 gram of sodium dodecyl benzene sulfonate. The head was placed on the flask and a thermocouple, nitrogen inlet, and mechanical stirrer attached. The contents were heated with infrared lamps to about 60° C while stirring at 350 rpm. A solution of 1.36 grams potassium persulfate in 20 grams deionized water was charged, the flask sealed, and a vacuum pulled on the flask four times, breaking it each time with nitrogen. The flask was held at 60° C for 20 minutes, then heated to 80° C over 10 minutes. Of the remaining amount of the first monomer solution, a pre-emulsion was prepared by charging a solution of 2 grams of sodium dodecyl benzene sulfonate in 80 grams of deionized water to the first monomer solution and stirring under nitrogen. This pre-emulsion was added dropwise to the two-liter flask at a rate of 6 grams per minute, the addition taking one hour. After the addition the reaction held at 80° C for thirty minutes.

A second pre-emulsion was prepared by adding a solution of 2.5 grams of sodium dodecyl benzene sulfonate in 121 grams of deionized water to a solution of 0.5 grams carbon tetrabromide in a mixture of 125 grams 2-EHA, 112.5 grams IBOA, and 12.5 grams AA and stirring under nitrogen. This second pre-emulsion was charged dropwise to the two liter flask over the course of 1.5 hours. After the addition, the stirring rate was reduced to 200 rpm and the reaction held at 80° C for two hours, then the resulting latex was filtered through doubled over cheesecloth into a jar. Low levels of coagulum were noted as a scum floating at the top of the reactor.

### Examples 13 to 16

### Copolymers made by solution polymerization and inversion in water

Into a 120 milliliter glass bottle was charged 24 grams of monomers (detailed in Table II below, all monomer amounts listed in grams), 120 milligrams of carbon tetrabromide, 36 grams of methylethyl ketone, and 72 milligrams of azobis(isobutyronitrile). The contents of the bottle were swept with nitrogen at about 1 liter per minute for two minutes, then the bottle was capped and tumbled in a water bath for 24 hours at about 55° C yielding a moderate viscosity solution. 15 grams (containing 6 grams of polymer or 8.3 milliequivalents of carboxylic acid) of the resulting solution was charged into a 250 milliliter round bottom flask containing a solution of 0.67 grams (7.5 milliequivalents, 90% neutralization) of 2-amino-2-methyl-1-propanol in 14 grams of deionized water with moderate agitation. The solvent was removed from the resulting dispersion by a rotary evaporator set at about 63° C at a reduced pressure of 40 kilopascals yielding a milky white dispersion. The resulting dispersions were coated as described above.

**Table II: Monomer Charges Used for Solution Polymerization and Inversion**

| **Example** | **2-EHA** | **IBOA** | **CHXMA** | **AA** | **T_{g} (°C)** | **Tack(g)** | **Flexibility** |
|---|---|---|---|---|---|---|---|
| 13 | 12.6 | 9.0 | 0 | 2.4 | -6 | 0 | Pass |
| 14 | 11.4 | 10.2 | 0 | 2.4 | -6 | 0 | Pass |
| 15 | 10.8 | 0 | 10.8 | 2.4 | 19 | 0 | Pass |
| 16 | 8.4 | 0 | 13.2 | 2.4 | 33 | 0 | Pass |
| Comparative G | 6.0 | 0 | 15.6 | 2.4 | 47 | 0 | Fail |

| | | | | | | | |
|---|---|---|---|---|---|---|---|
| CHXMA = cyclohexyl methacrylate | | | | | | | |

### Example 17 to 19, Comparative Example H

### Copolymers made by suspension polymerization

In a one liter Mortonized split resin flask was charged 240 grams of a monomer mixture (detailed in Table III, all monomer amounts listed in grams). Added to the flask was 6.9 grams Ludox^{™} 50 (50% by wt colloidal silica in water, available from Aldrich, Milwaukee, WI), 360 grams deionized water, 0.42 grams adipic acid/diethanol amine condensate (a 50% solids used as a promoter, prepared according to the procedure disclosed in U.S. Patent No. 5,238,736), and 0.08 grams potassium dichromate. The head was placed on the flask and a thermocouple, nitrogen inlet, and mechanical stirrer attached. The entire content inside the flask is mixed. The pH is measured and adjusted by adding ammonium hydroxide to a pH between 4 and 5. The mixture was then transferred to a Waring^{™} blender and exposed to high shear (about 22,000 rpm) for six minutes total, using shear for about two minutes at a time to avoid overheating the mixture.

The mixture was then returned to the Mortonized flask and 0.36 grams of Vazo^{™} 64 (azo-bis(isobutyronitrile) initiator, available from E.I. du Pont de Nemours & Co., Wilmington, DE) was added. A nitrogen purge was started and the mixture is agitated gently for several minutes to let the initiator dissolve. The agitation speed is adjusted to about 300 rpm and the temperature was set at about 60° C. The reaction started within minutes and was allowed to exotherm. After exotherming, the temperature was maintained at about 60° C for about four hours.

**Table III: Monomer Charges Used for Suspension Polymerization**

| **Example** | **Parts** | **2-EHA** | **IBOA** | **MAA** | **T_{g} (°C)** | **Tack (g)** | **Flexibility** |
|---|---|---|---|---|---|---|---|
| Comparative H | 70/25/5 | 168 | 60 | 12 | -41 | 135 | Pass |
| 17 | 50/45/5 | 120 | 108 | 12 | -14 | 0 | Pass |
| 18 | 55/40/5 | 132 | 96 | 12 | -22 | 0 | Pass |
| 19 | 60/35/5 | 144 | 84 | 12 | -25 | 0 | Pass |

The "Parts" column indicates the parts by weight of the 2-EHA/IBOA/MAA components. Thus, for Example 17, of the 240 grams of the monomer mixture, 50 parts by weight was 2-EHA, translating to 120 grams. The data in Table III shows that Comparative Example H had unacceptably high tack.

### Example 20

### Cosmetic example of body lotions

An oil-in-water body lotion was prepared from the emulsion polymer of Example 1 as follows. In separate vessels the components of Phase A and Phase B in Table IV were heated to about 70° C with mixing. Phase B was added to phase A and homogenized using a high shear mixer. After cooling, a substantive, non-greasy, non-tacky body lotion resulted. Body lotions from the emulsion polymers of Examples 2, 6, 7, 8, 16, and 18 were prepared in similar fashion by replacing Example 1 emulsion polymer with the appropriate polymers from Examples 2, 6, 7, 8, 16, and 18. The percentages in Table IV are weight percent of the total lotion composition.

**Table IV: Oil-in-Water Body Lotion**

| **Phase A** | |
|---|---|
| Mineral Oil | 10% |
| Isopropyl myristate | 2% |
| Glyceryl stearate | 3% |
| Stearic acid | 4% |
| Ceteth 20 | 1% |
| Lanolin oil | 0.6% |
| | |

| **Phase B** | |
|---|---|
| Deionized water | 73% |
| Ex. 1 Emulsion Polymer | 5% |
| HEC | 0.2% |
| Triethanol amine | 1.2% |

### Examples 21 and 22

### Cosmetic examples of moisturizing foundation

An oil-in-water foundation was prepared from the emulsion polymer of Example 12 or a one-to-one blend of the emulsions from Examples 10 and 11 as follows. A pigment masterbatch was prepared by milling 80 parts of titanium dioxide with 9.5 parts of yellow iron oxide, 9.5 parts of red iron oxide, 0.7 parts of black iron oxide, and 42.3 parts of talc. In separate vessels the components of Phase A and Phase B in Table V were heated to 75° C with mixing. Phase A was added to phase B and homogenized using a high shear mixer. Cooling under low agitation yields a creamy moisturizing foundation.

**Table V: Oil-in-Water Foundation**

| **Phase A** | **Example 21** | **Example 22** |
|---|---|---|
| Mineral oil | 9.4% | 9.4% |
| Isopropyl myristate | 4% | 4% |
| Glyceryl stearate | 2% | 2% |
| Stearic acid | 2.6% | 2.6% |
| | | |

| **Phase B** | | |
|---|---|---|
| Pigment master batch | 14% | 14% |
| Deionized water | 56.2% | 56.2% |
| Ex. 12 Emulsion Polymer | 8% | |
| Ex. 10 Emulsion Polymer | | 4% |
| Ex. 11 Emulsion Polymer | | 4% |
| Lecithin | 2% | 2% |
| Magnesium aluminum silicate | 0.4% | 0.4% |
| HEC | 0.4% | 0.4% |
| Triethanol amine | 1% | 1% |

### Example 23

### Cosmetic example of mascara

An oil-in-water mascara was prepared from the emulsion polymer of Example 4 as follows. In separate vessels the components of Phase A and Phase B in Table V were heated to 70° C with mixing. Phase B was added to phase A and homogenized using a high shear mixer. After cooling, a flake-, smudge-, and water-resistant mascara results. Mascara from the emulsion polymers of Example 8 and Comparative Examples A and B were prepared in similar fashion as was a control with water in place of the emulsion polymer.

**Table VI: Oil-in-Water Mascara**

| **Phase A** | |
|---|---|
| Carnuba Wax | 10% |
| Isopropyl myristate | 6% |
| Glyceryl stearate | 3% |
| Stearic acid | 5% |
| Black iron oxide | 10% |
| | |

| **Phase B** | |
|---|---|
| Deionized water | 43.5% |
| Ex. 4 Emulsion Polymer | 20% |
| PVP | 1% |
| HEC | 0.2% |
| Triethanol amine | 1.3% |

A portion of each mascara formulation was coated with a knife coater onto 0.0015 inch (38 micrometers) polyester film to a dry coating thickness of about 0.002 inch (51 micrometers). After drying at room temperature for about 24 hours, the coatings were qualitatively assessed for smudge resistance, tack, flake resistance, and water resistance. Smudge resistance was judged by rubbing with a finger and seeing how much had transferred to the finger. Tack was judged by pressing a finger down briefly and removing it, seeing how strong a bond was formed to the coating. Flake resistance was assessed by bending and creasing the polyester film and observing if the mascara coating cracked off the film. Water resistance was judged by suspending a 1 inch strip of the coated film in an agitated 32°C water bath for about 20 minutes, then assessing the smudge resistance of the still wet coating. Examples 4 and 8 and Comparative B formed balls of coating on rubbing in this test, suggesting that the coating integrity was still good, but the adhesion of the coating to the polyester had been compromised by the water. Results are shown in Table VI below.

**Table VII: Qualitative Testing of Mascara Performance**

| **Polymer Used** | **Transfer** | **Tack** | **Flake** | **Wet Transfer** |
|---|---|---|---|---|
| None | A lot | Low | Some | Complete |
| Example 4 | None | Low | None | Balls up |
| Example 8 | None | Low | None | Balls up |
| Comparative A | Some | Moderate | None | Some |
| Comparative B | Some | Moderate | None | Balls up |

Data for Examples 4 and 8 showed that they have all the desirable features for a mascara application. The control sample containing no polymer had unacceptably high amount of transfer. Comparative examples A and B also showed some transfer and moderate tack.

## Claims

1. Use of a composition in the form of an aqueous emulsion or dispersion, said composition comprising:
(a) at least one copolymer comprising (i) 10 to 85 weight percent of (meth)acrylate ester of C₄ to C₁₈ straight and/or branched chain alkyl alcohol, (ii) 10 to 70 weight percent of (meth)acrylate ester of a saturated or unsaturated cyclic alcohol containing 6 to 20 carbon atoms; and
(b) an aqueous carrier, solvent, or vehicle component,
in cosmetic and personal care applications, selected from the group consisting of mascara, foundation, rouge, face powder, eyeliner, eyeshadow, lipstick, insect repellent, nail polish, skin moisturizer, skin cream, body lotion sunscreen.

2. The use of claim 1, wherein said (a)(i) component is selected from the group consisting of isooctyl (meth)acrylate, n-butyl (meth)acrylate, isobutyl acrylate, t-butyl (meth)acrylate, 2-methylbutyl acrylate, 2-ethylhexyl (meth)acrylate, n-octyl (meth)acrylate, isononyl (meth)acrylate, lauryl (meth)acrylate, octadecyl (meth)acrylate, and combinations thereof.

3. The use of claim 1, wherein said (a)(ii) component is selected from the group consisting of bicyclo[2.2.1]heptyl (meth)acrylate; adamantyl (meth)acrylate; 3,5-dimethyladamantyl (meth)acrylate; isobornyl (meth)acrylate; tolyl (meth)acrylate; phenyl (meth)acrylate; t-butylphenyl (meth)acrylate; 2-naphthyl (meth)acrylate; benzyl methacrylate; cyclohexyl methacrylate; menthyl methacrylate; 3,3,5-trimethylcyclohexyl methacrylate; dicyclopentenyl (meth)acrylate; 2-(dicyclopentenyloxy)ethyl (meth)acrylate; and combinations thereof.

4. The use of claim 1 wherein said copolymer further comprises up to about 20 weight percent of a hydrophilic monomer.

5. The use of claim 4, wherein said hydrophilic monomer is selected from the group consisting of acrylic acid, methacrylic acid, N-vinyl-2-pyrrolidone and combintaions thereof.

6. The use of claim 1, wherein said composition is formed into a film, said film having less than about 50 grams of tack when tested according to ASTM D 2979-95.

7. The use of claim 1, wherein said composition is formed into a film, said film passes the flexibility test when tested according to ASTM D 4338-97.

8. The use of claim 1, wherein said copolymer has average particle size of less than about 1 micrometers.

9. The use of claim 1 wherein said composition has a T_{g} less than 35° C.

10. The use of claim 1 wherein said composition further comprises ingredients selected from the group consisting of emollients, humectants, propellants, pigments, dyes, buffers, organic suspending agents, inorganic suspending agents, organic thickening agents, inorganic thickening agents, waxes, surfactants, plasticizers, preservatives, flavoring agents, perfumes, vitamins, herbal extracts, skin bleaching agents, hair bleaching agents, skin coloring agents, hair coloring agents, antimicrobial agents, and antifungal agents and combinations thereof.

11. The use of claim 1 wherein said composition comprises a blend of said copolymer.

12. The use of claim 1, wherein said (b) component is selected from the group consisting of water, C₁ to C₄ branched or straight chain aliphatic alcohol, and combinations thereof.

13. The use of claim 12, wherein said C₁ to C₄ branched or straight chain aliphatic alcohol is selected from the group consisting of ethanol, n-propanol, 2-propanol, and combinations thereof.

14. The use of claim 12 wherein said composition further comprises solvents selected from the group consisting of hexamethyldisiloxane, cyclic silicones, C₄ to C₁₀ alkanes, acetone, and hydrofluoroethers.

## Patentansprüche

1. Verwendung einer Zusammensetzung in Form einer wässrigen Emulsion oder Dispersion, wobei die Zusammensetzung Folgendes umfasst:
(a) mindestens ein Copolymer, umfassend (i) 10 bis 85 Gewichtsprozent (Meth)acrylatester eines gerad- und/oder verzweigtkettigen C₄- bis C₁₈-Alkylalkohols, (ii) 10 bis 70 Gewichtsprozent (Meth)acrylatester eines gesättigten oder ungesättigten cyclischen Alkohols mit 6 bis 20 Kohlenstoffatomen; und
(b) eine wässrige Träger-, Lösungsmittel- oder Vehikelkomponente,
in Kosmetik- und Körperpflegeanwendungen, die ausgewählt sind aus der Gruppe, die aus Wimperntusche, Grundierung, Rouge, Gesichtspuder, Eyeliner, Lidschatten, Lippenstift, Insektenabwehrmittel, Nagellack, Hautfeuchtigkeitscreme, Hautcreme, Körperlotion und Sonnenschutzmittel besteht.

2. Verwendung nach Anspruch 1, wobei die Komponente (a) (i) ausgewählt ist aus der Gruppe, die aus Isooctyl(meth)acrylat, n-Butyl(meth)acrylat, Isobutylacrylat, t-Butyl(meth)acrylat, 2-Methylbutylacrylat, 2-Ethylhexyl(meth)acrylat, n-Octyl(meth)acrylat, Isononyl(meth)acrylat, Lauryl(meth)acrylat, Octadecyl(meth)acrylat und Kombinationen davon besteht.

3. Verwendung nach Anspruch 1, wobei die Komponente (a) (ii) ausgewählt ist aus der Gruppe, die aus Bicyclo[2.2.1]heptyl(meth)acrylat; Adamantyl(meth)acrylat; 3,5-Dimethyladamantyl(meth)acrylat; Isobornyl(meth)acrylat; Tolyl(meth)acrylat; Phenyl(meth)acrylat; t-Butylphenyl(meth)acrylat; 2-Naphthyl(meth)acrylat; Benzylmethacrylat; Cyclohexylmethacrylat; Menthylmethacrylat; 3,3,5-Trimethylcyclohexylmethacrylat; Dicyclopentenyl(meth)acrylat; 2-(Dicyclopentenyloxy)ethyl(meth)acrylat und Kombinationen davon besteht.

4. Verwendung nach Anspruch 1, wobei das Copolymer ferner bis zu etwa 20 Gewichtsprozent eines hydrophilen Monomers umfasst.

5. Verwendung nach Anspruch 4, wobei das hydrophile Monomer ausgewählt ist aus der Gruppe, die aus Acrylsäure, Methacrylsäure, N-Vinyl-2-pyrrolidon und Kombinationen davon besteht.

6. Verwendung nach Anspruch 1, wobei die Zusammensetzung zu einem Film geformt ist, wobei der Film bei der Prüfung gemäß ASTM D 2979-95 eine Klebrigkeit von weniger als etwa 50 Gramm aufweist.

7. Verwendung nach Anspruch 1, wobei die Zusammensetzung zu einem Film geformt ist, wobei der Film bei der Prüfung gemäß ASTM D 4338-97 die Flexibilitätsprüfung besteht.

8. Verwendung nach Anspruch 1, wobei das Copolymer eine mittlere Teilchengröße von weniger als etwa 1 Mikrometer aufweist.

9. Verwendung nach Anspruch 1, wobei die Zusammensetzung eine T_{g} von weniger als 35 °C aufweist.

10. Verwendung nach Anspruch 1, wobei die Zusammensetzung ferner Bestandteile umfasst, die ausgewählt sind aus der Gruppe, die aus Emollientien, Feuchthaltemitteln, Treibmitteln, Pigmenten, Farbstoffen, Puffern, organischen Suspensionsmitteln, anorganischen Suspensionsmitteln, organischen Verdickungsmitteln, anorganischen Verdickungsmitteln, Wachsen, Tensiden, Weichmachern, Konservierungsmitteln, Geschmacksstoffen, Duftstoffen, Vitaminen, Kräuterextrakten, Hautbleichmitteln, Haarbleichmitteln, Hautfärbemitteln, Haarfärbemitteln, antimikrobiellen Mitteln und antifungalen Mitteln und Kombinationen davon besteht.

11. Verwendung nach Anspruch 1, wobei die Zusammensetzung eine Mischung des Copolymers umfasst.

12. Verwendung nach Anspruch 1, wobei die Komponente (b) ausgewählt ist aus der Gruppe, die aus Wasser, verzweigt- oder geradkettigem aliphatischem C₁- bis C₄-Alkohol und Kombinationen davon besteht.

13. Verwendung nach Anspruch 12, wobei der verzweigt- oder geradkettige aliphatische C₁- bis C₄-Alkohol ausgewählt ist aus der Gruppe, die aus Ethanol, n-Propanol, 2-Propanol und Kombinationen davon besteht.

14. Verwendung nach Anspruch 12, wobei die Zusammensetzung ferner Lösungsmittel umfasst, die ausgewählt sind aus der Gruppe, die aus Hexamethyldisiloxan, cyclischen Silikonen, C₄- bis C₁₀-Alkanen, Aceton und Hydrofluorethern besteht.

## Revendications

1. Utilisation d'une composition sous la forme d'une émulsion ou dispersion aqueuse, ladite composition comprenant :
(a)au moins un copolymère comprenant (i) de 10 à 85 pour cent en poids d'un ester (méth)acrylate d'un alcanol en C₄ à C₁₈ à chaîne linéaire et/ou ramifiée, (ii) de 10 à 70 pour cent en poids d'un ester (méth)acrylate d'un alcool cyclique saturé ou insaturé contenant de 6 à 20 atomes de carbone ; et
(b)un véhicule, solvant ou vecteur aqueux,
dans des applications de cosmétique et de soins personnels choisies dans le groupe constitué par un mascara, un fond de teint, un fard à joues, une poudre de riz, un eye-liner, une ombre à paupières, un rouge à lèvres, un insectifuge, un vernis à ongles, un hydratant pour la peau, une crème pour la peau, une lotion pour le corps et un écran solaire.

2. Utilisation selon la revendication 1, dans laquelle ledit constituant (a)(i) est choisi dans le groupe constitué par le (méth)acrylate d'isooctyle, le (méth)acrylate de n-butyle, l'acrylate d'isobutyle, le (méth)acrylate de t-butyle, l'acrylate de 2-méthylbutyle, le (méth)acrylate de 2-éthylhexyle, le (méth)acrylate de n-octyle, le (méth)acrylate d'isononyle, le (méth)acrylate de lauryle, le (méth)acrylate d'octadécyle et leurs combinaisons.

3. Utilisation selon la revendication 1, dans laquelle ledit constituant (a)(ii) est choisi dans le groupe constitué par le (méth)acrylate de bicyclo[2.2.1]heptyle, le (méth)acrylate d'adamantyle, le (méth)acrylate de 3,5-diméthyladamantyle, le (méth)acrylate d'isobornyle, le (méth)acrylate de tolyle, le (méth)acrylate de phényle, le (méth)acrylate de t-butylphényle, le (méth)acrylate de 2-naphtyle, le méthacrylate de benzyle, le méthacrylate de cyclohexyle, le méthacrylate de menthyle, le méthacrylate de 3,3,5-triméthylcyclohexyle, le (méth)acrylate de dicyclopentényle, le (méth)acrylate de 2-(dicyclopentényloxy)éthyle et leurs combinaisons.

4. Utilisation selon la revendication 1, dans laquelle ledit copolymère comprend en outre jusqu'à environ 20 pour cent en poids d'un monomère hydrophile.

5. Utilisation selon la revendication 4, dans laquelle ledit monomère hydrophile est choisi dans le groupe constitué par l'acide acrylique, l'acide méthacrylique, la N-vinyl-2-pyrrolidone et leurs combinaisons.

6. Utilisation selon la revendication 1, dans laquelle ladite composition est mise sous forme d'un film, ledit film présentant moins d'environ 50 grammes de poisseux quand on effectue l'essai selon ASTM D 2979-95.

7. Utilisation selon la revendication 1, dans laquelle ladite composition est mise sous forme d'un film, ledit film réussissant l'essai de flexibilité quand on effectue l'essai selon ASTM D 4338-97.

8. Utilisation selon la revendication 1, dans laquelle ledit copolymère a une granulométrie moyenne inférieure à environ 1 micromètre.

9. Utilisation selon la revendication 1, dans laquelle ladite composition possède une Tᵥ inférieure à 35 °C.

10. Utilisation selon la revendication 1, dans laquelle ladite composition comprend en outre des ingrédients choisis dans le groupe constitué par les émollients, les humectants, les propulseurs, les pigments, les colorants, les tampons, les agents de mise en suspension organiques, les agents de mise en suspension inorganiques, les agents épaississants organiques, les agents épaississants inorganiques, les cires, les tensioactifs, les plastifiants, les conservateurs, les agents aromatisants, les parfums, les vitamines, les extraits d'herbes, les agents de blanchiment de la peau, les agents de blanchiment des cheveux, les agents de coloration de la peau, les agents de coloration des cheveux, les agents antimicrobiens et les agents antifongiques et leurs combinaisons.

11. Utilisation selon la revendication 1, dans laquelle ladite composition comprend un mélange dudit copolymère.

12. Utilisation selon la revendication 1, dans laquelle ledit constituant (b) est choisi dans le groupe constitué par l'eau, un alcool aliphatique en C₁ à C₄ à chaîne ramifiée ou linéaire et leurs combinaisons.

13. Utilisation selon la revendication 12, dans laquelle ledit alcool aliphatique en C₁ à C₄ à chaîne ramifiée ou linéaire est choisi dans le groupe constitué par l'éthanol, le n-propanol, le 2-propanol et leurs combinaisons.

14. Utilisation selon la revendication 12, dans laquelle ladite composition comprend en outre des solvants choisis dans le groupe constitué par l'hexaméthyldisiloxane, les silicones cycliques, les alcanes en C₄ à C₁₀, l'acétone et les hydrofluoroéthers.
